# EUROPEAN PATENT SPECIFICATION

(11) **EP 0 906 258 B1**
(45) Date of publication and mention of the grant of the patent: **13.03.2002**
(21) Application number: 97928152.4
(22) Date of filing: 29.05.1997
(51) Int. Cl.: C07C 29/141, C07C 31/20, C07C 45/58, C07C 47/19

(54) **PROCESS FOR PREPARING 1,3-ALKANEDIOLS**
VERFAHREN ZUR HERSTELLUNG VON 1,3-ALKANDIOLEN
PROCEDE DE PREPARATION DE 1,3-ALCANEDIOLS

(30) Priority: 30.05.1996 US 655639; 23.07.1996 US 685173; 15.10.1996 US 729932; 15.10.1996 US 729971
(43) Date of publication of application: 07.04.1999
(73) Proprietor: SHELL INTERNATIONALE RESEARCH MAATSCHAPPIJ B.V., 2596 HR Den Haag (NL)
(72) Inventor: ARHANCET, Juan, Pedro, Katy, TX 77450 (US); MATZAKOS, Andreas, Nikolaos, Missouri City, TX 77459 (US); PLEDGER, William, Ridley, Pearland, TX 77581 (US); POWELL, Joseph, Broun, Houston, TX 77070 (US); WEIDER, Paul, Richard, Houston, TX 77083 (US); SLAUGH, Lynn, Henry, Houston, TX 77070 (US)
(86) International application number: EP9702953
(87) International publication number: WO9745390

(56) References cited:
- WO-A-96/10552
- US-A- 3 687 981
- US-A- 5 015 789
- US-A- 5 256 827

## Description

This invention relates to the preparation of 1,3-alkanediols. In a specific aspect, the invention relates to the preparation of 1,3-propanediol from ethylene oxide via hydrogenation of 3-hydroxypropanal.

1,3-propanediol (PDO) is an intermediate in the production of polyesters for fibers and films. It is known to prepare PDO in a process involving (1) the hydrogenation of 3-hydroxypropanal (HPA). HPA may be prepared by hydroformylation (reaction with synthesis gas, H₂/CO) of ethylene oxide. HPA may also be prepared from. e.g., glycerin and acrolein.

For process economics, it is important for the HPA hydrogenation step to be highly selective to PDO. Selectivity is, however, complicated by the high reactivity of HPA, which can react with species in the hydrogenation reaction mixture to form by-products which lower product yield and complicate product recovery. It is known to improve selectivity by using a slurry catalyst at relatively low temperatures between 60 and 90 °C. Slurry catalyst systems are, however, quite costly to operate.

Valerius et al, in Chemical Engineering and Processing 35 (1996) 11-19, already illustrated the hydrogenation of an aqueous 10 wt% solution of HPA cocurrently with hydrogen through a trickle bed. However, when this example and others at slightly higher concentration were repeated, unattractive results were obtained.

It would therefore be desirable to prepare PDO from HPA with high selectivity and activity in a relatively inexpensive fixed-bed catalyst system.

It is therefore an object of the invention to provide a selective process for the preparation of 1,3-alkanediols including PDO from 3-hydroxyalkanals including HPA using a fixed-bed hydrogenation catalyst.

According to the invention, 1,3-alkanediols including 1,3-propanediol are prepared via the hydrogenation of 3-hydroxyalkanals wherein the process comprises passing an aqueous 3-hydroxyalkanal solution to a hydrogenation zone and in contact with a fixed-bed hydrogenation catalyst under hydrogenation conditions to form an aqueous 1,3-alkanediol solution, characterized in that the aqueous 3-hydroxyalkanal solution has a 3-hydroxyalkanal concentration within the range of 0.2 to 5 wt%.

According to a preferred embodiment of the invention, 1,3-propanediol is prepared in a process comprising the steps of:
(a) contacting, at a temperature within the range of 50 to 100 °C and a pressure within the range of 0.7 to 34.5 MPa (500 to 5000 psig), ethylene oxide with carbon monoxide and hydrogen in an essentially non-water-miscible solvent in the presence of an effective amount of a non-phosphine-ligated cobalt or rhodium catalyst and an effective amount of a catalyst promoter under reaction conditions effective to produce an intermediate product mixture comprising less than 15 weight percent (wt%) 3-hydroxypropanal;
(b) adding an aqueous liquid to said intermediate product mixture and extracting into said aqueous liquid a major portion of the 3-hydroxypropanal at a temperature less than 100 °C so as to provide an aqueous phase comprising 3-hydroxypropanal in a concentration of at least 20 wt%, and an organic phase comprising at least a portion of the cobalt or rhodium catalyst or a cobalt- or rhodium-containing derivative thereof;
(c) separating the aqueous phase from the organic phase;
(d) diluting said aqueous phase with an aqueous liquid to form a 3-hydroxypropanal solution having a 3-hydroxypropanal concentration within the range of 0.2 to 5 wt%;
(e) passing said aqueous 3-hydroxypropanal solution to a hydrogenation zone and in contact with a fixed-bed hydrogenation catalyst under hydrogenation conditions to form an aqueous 1,3-propanediol; and
(f) recovering said 1,3-propanediol.

In a specific embodiment of this process, dilution of the relatively concentrated 3-hydroxypropanal solution is effected with an aqueous 1,3-propanediol solution passed to the 3-hydroxypropanal solution from downstream hydrogenation. Dilution of this aqueous 3-hydroxypropanal solution prior to hydrogenation permits the use of a relatively inexpensive fixed-bed hydrogenation catalyst without sacrificing hydrogenation selectivity. Use of an aqueous solution of 1,3-propanediol to dilute the 3-hydroxypropanal solution further enhances selectivity and yields of 1,3-propanediol, prolongs catalyst life, and saves on equipment and cooling costs.

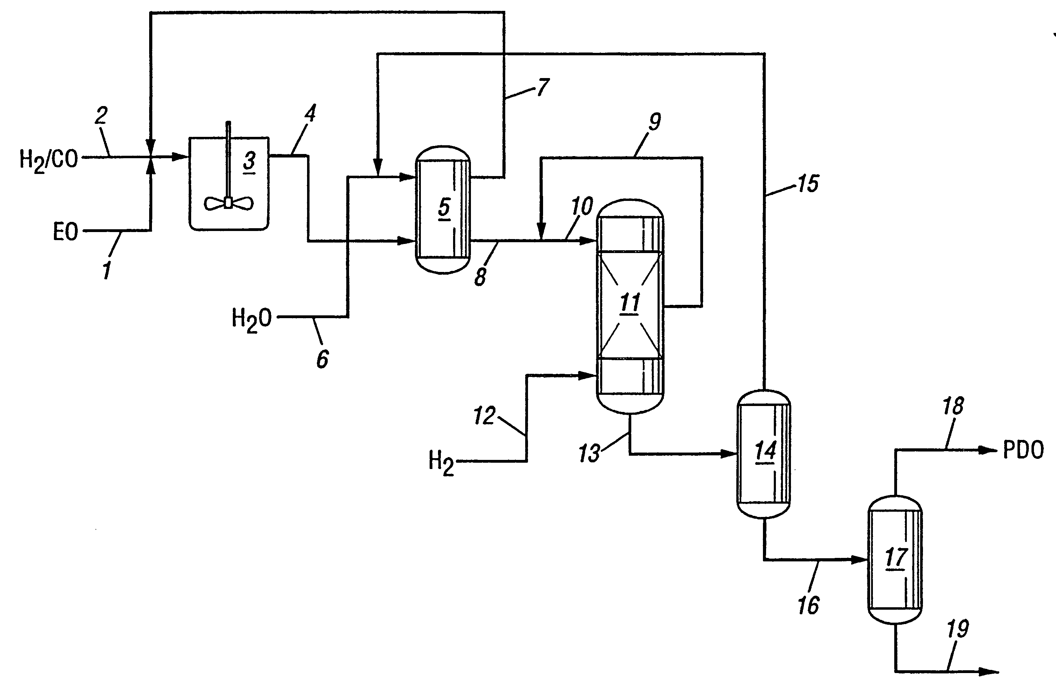

The invention can be conveniently described by reference to the Figure, which illustrates an embodiment of the invention process for preparation of 1,3-propanediol by hydroformylation of ethylene oxide to 3-hydroxypropanal and then hydrogenation of the 3-hydroxypropanal to 1,3-propanediol.

In the illustrated embodiment, separate or combined streams of ethylene oxide 1, carbon monoxide and hydrogen 2 are charged to hydroformylation vessel 3, which can be a pressure reaction vessel such as a bubble column or agitated tank, operated batchwise or in a continuous manner. The feed streams are contacted in the presence of a hydroformylation catalyst, generally a metal carbonyl selected from rhodium and cobalt carbonyls. The hydroformylation catalyst will generally be present in the reaction mixture in an amount within the range of 0.01 to 1 wt%, preferably 0.05 to 0.3 wt%, based on the weight of the hydroformylation reaction mixture. The hydrogen and carbon monoxide will generally be introduced into the reaction vessel in a molar ratio within the range of 1:2 to 8:1, preferably 1:1 to 6:1.

The hydroformylation reaction is carried out under conditions effective to produce a hydroformylation reaction product mixture containing a major portion of 3-hydroxypropanal and a minor portion of acetaldehyde and 1,3-propanediol, while maintaining the level of 3-hydroxypropanal in the reaction mixture at less than 15 wt%, preferably within the range of 5 to 10 wt%. (To provide for solvents having different densities, the desired concentration of 3-hydroxypropanal in the reaction mixture can be expressed in molarity, i.e., less than 1.5M, preferably within the range of 0.5 to 1M). Generally, the cobalt-catalyzed hydroformylation reaction is carried out at elevated temperature less than 100 °C, preferably 60 to 90 °C, most preferably 75 to 85 °C, with rhodium-catalyzed hydroformylations on the order of 10 °C higher. The hydroformylation reaction is generally carried out at a pressure within the range of 0.7 to 34.5 MPa (100 to 5000 psig), preferably (for process economics) 6.9 to 24.1 (1000 to 3500 psig), with higher pressures preferred for greater selectivity. The concentration of 3-hydroxypropanal in the intermediate product mixture can be controlled by regulation of process conditions such as ethylene oxide concentration, catalyst concentration, reaction temperature and residence time. In general, relatively low reaction temperatures (below 90 °C) and relatively short residence times (20 minutes to 1 hour) are preferred.

The hydroformylation reaction is carried out in a liquid solvent inert to the reactants. By "inert" is meant that the solvent is not consumed during the course of the reaction. In general, ideal solvents for the hydroformylation process will solubilize carbon monoxide, will be essentially non-water-miscible and will exhibit low to moderate polarity such that the 3-hydroxypropanal intermediate will be solubilized to the desired concentration of at least 5 wt% under hydroformylation conditions, while significant solvent will remain as a separate phase upon water extraction. By "essentially non-water-miscible" is meant that the solvent has a solubility in water at 25 °C of less than 25 wt%, so as to form a separate hydrocarbon-rich phase upon water extraction of 3-hydroxypropanal from the hydroformylation reaction mixture. Preferably this solubility is less than 10 wt%, most preferably less than 5 wt%. The solubilization of carbon monoxide in the selected solvent will generally be greater than 0.15 v/v (1 atm, 25 °C), preferably greater than 0.25 v/v, as expressed in terms of Ostwald coefficients.

The preferred class of solvents are alcohols and ethers which can be described according to the formula

R₂-O-R₁ (1)

in which R₁ is hydrogen or C₁₋₂₀ linear, branched, cyclic or aromatic hydrocarbyl or mono- or polyalkylene oxide, and R₂ is C₁₋₂₀ linear, branched, cyclic or aromatic hydrocarbyl, alkoxy or mono- or polyalkylene oxide. The most preferred hydroformylation solvents can be described by the formula 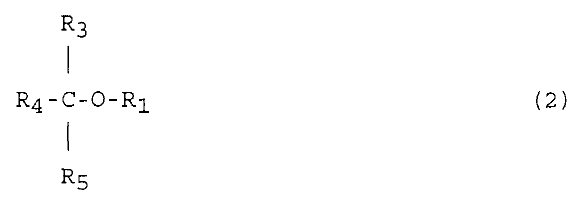 in which R₁ is hydrogen or C₁₋₈ hydrocarbyl, and R₃, R₄ and R₅ are independently selected from C₁₋₈ hydrocarbyl, alkoxy and alkylene oxide. Such ethers include, for example, methyl-t-butyl ether, ethyl-t-butyl ether, diethyl ether, phenylisobutyl ether, ethoxyethyl ether, diphenyl ether and diisopropyl ether. Blends of solvents such as tetrahydrofuran/toluene, tetrahydrofuran/heptane and t-butylalcohol/hexane can also be used to achieve the desired solvent properties. The currently preferred solvent, because of the high yields of 3-hydroxypropanal which can be achieved under moderate reaction conditions, is methyl-t-butyl ether.

To further enhance yields under moderate reaction conditions, the hydroformylation reaction mixture will preferably include a catalyst promoter to accelerate the reaction rate. Suitable promoters include sources of mono- and multivalent metal cations of weak bases such as alkali, alkaline earth and rare earth metal salts of carboxylic acids. Also suitable are lipophilic promoters such as lipophilic phosphonium salts, lipophilic amines and lipophilic ruthenium catalyst promoters, which accelerate the rate of hydroformylation without imparting hydrophilicity (water solubility) to the active catalyst. As used herein, "lipophilic" means that the promoter tends to remain in the organic phase after extraction of 3-hydroxypropanal with water. The promoter will generally be present in an amount within the range of 0.001 to 1.0 mole per mole of cobalt or rhodium. Suitable metal salts include sodium, potassium and cesium acetates, propionates and octoates; calcium carbonate and lanthanum acetate. The preferred ruthenium source is triruthenium dodecacarbonyl, available commercially from Aldrich Chemical Company, which is used in an amount within the range of 0.001 to 0.5 mole per mole of cobalt, preferably 0.008 to 0.1 mole/mole. The currently preferred lipophilic promoters are tetrabutylphosphonium acetate and dimethyldodecyl amine. Alternative promoters include substituted and unsubstituted porphyrines according to the formula 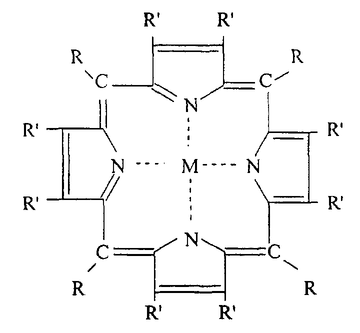 in which M is a transition metal, but preferably manganese, cobalt or rhodium, each of R and R' is selected independently from hydrogen, halide, alkoxy, aryloxy, and substituted or unsubstituted hydrocarbyl including alkyl and aryl groups. The preferred porphyrines, because of their commercial availability and demonstrated effectiveness, are 5,10,15,20-tetraphenyl-21H,23H-porphyrine cobalt(II), CAS#[14172-90-8] and 5,10,15,20-tetraphenyl-21H,23H-porphyrine manganese(III) chloride. The transition metal will generally be present in the promoter in an amount within the range of about 0.001 to about 0.5 mole per mole of cobalt or rhodium metal in the hydroformylation catalyst, preferably about 0.008 to about 0.1 mole. Larger amounts of the porphyrine promoter can be used but are not necessary for promotional effect.

It is generally preferred to regulate the concentration of water in the hydroformylation reaction mixture, as excessive amounts of water reduce (HPA + PDO) selectivity below acceptable levels and may induce formation of a second liquid phase. At low concentrations, water can assist in promoting the formation of the desired cobalt carbonyl catalyst species. Acceptable water levels will depend upon the solvent used, with more polar solvents generally being more tolerant of higher water concentrations. For example, optimum water levels for hydroformylation in methyl-t-butyl ether solvent are believed to be within the range of 1 to 2.5 wt%.

Following the hydroformylation reaction, hydroformylation reaction product mixture 4 containing 3-hydroxypropanal, the reaction solvent, 1,3-propanediol, the catalyst and a minor amount of reaction by-products, is cooled and passed to extraction vessel 5, wherein an aqueous liquid, generally water and optional miscibilizing solvent, are added via 6 for extraction and concentration of the 3-hydroxypropanal for the subsequent hydrogenation step. Liquid extraction can be effected by any suitable means, such as mixer-settlers, packed or trayed extraction columns, or rotating disk contactors. Extraction can, if desired, be carried out in multiple stages. The amount of water added to the hydroformylation reaction product mixture will generally be such as to provide a water:mixture ratio within the range of 1:1 to 1:20, preferably 1:5 to 1:15. Water extraction is preferably carried out at a temperature within the range of 25 to 55 °C, with lower temperatures preferred. Water extraction under 0.3 to 1.4 MPa (50 to 200 psig) carbon monoxide at 25 to 55 °C maximizes catalyst recovery in the organic phase.

The organic phase containing the reaction solvent and the major portion of the catalyst can be recycled, with optional purge of heavy ends, from the extraction vessel to hydroformylation reaction via 7. Aqueous extract 8 can optionally be subjected to additional operations, such as passage through an acid ion exchange resin bed, re-extraction with a non-water-miscible solvent, complete or partial oxidation of the catalyst metal followed by precipitation and filtration, deposition on a solid support, or extraction using a non-water-miscible solvent for removal of residual catalyst. Aqueous extract 8 is passed to hydrogenation zone 11 and reacted with hydrogen 12 in the presence of a hydrogenation catalyst to produce a hydrogenation product mixture 13 containing 1,3-propanediol.

In accordance with the invention process, input stream 10 to hydrogenation vessel 11 is an aqueous solution containing 3-hydroxypropanal in a concentration within the range of 0.2 to 5 wt%, preferably 0.3 to 4.8 wt%, based on the weight of the aqueous liquid. In the embodiment shown, outlet stream 8 from aqueous extraction of the hydroformylation product contains 3-hydroxypropanal in greater concentration (generally 20 to 40 wt%) than that desired for selective hydrogenation. Dilution is accomplished by addition of an aqueous liquid to this relatively concentrated 3-hydroxypropanal solution.

Although any aqueous liquid which will not interfere with hydrogenation of the 3-hydroxypropanal, including water, can be used for dilution of the 3-hydroxypropanal solution to the desired concentration, it is preferred to employ an aqueous 1,3-propanediol-containing solution such as hydrogenation output stream 9. Dilution with such a PDO-containing solution serves to concentrate PDO in the system water, thus avoiding the high cost of recovery of dilute PDO from water which would result from the use of water alone as the diluent. The preferred dilution stream will contain 1,3-propanediol and 3-hydroxypropanal in an amount within the range of 20 to 40 wt%, such as could be conveniently routed from an early stage of hydrogenation. The dilution stream 9 will preferably be cooled prior to admixture with the hydroformylation output stream to bring the temperature of the combined stream to that desired for input to the initial stage of hydrogenation.

In the illustrated embodiment, aqueous 1,3-propanediol stream 9 is used to dilute aqueous 3-hydroxypropanal stream 8. Other configurations of the hydrogenation input and recycle streams can be employed within the concept of the use of a 1,3-propanediol-containing stream for dilution of the 3-hydroxypropanal. For example, stream 8 can be divided for input into both a first hydrogenation catalyst bed and a second hydrogenation catalyst bed downstream from the first. The aqueous product stream passing from the first catalyst bed into the downstream second bed would serve to dilute the 3-hydroxypropanal feed into this second catalyst bed.

Hydrogenation of the aqueous 3-hydroxypropanal to 1,3-propanediol is carried out over a fixed-bed supported hydrogenation catalyst. The catalyst can be a Group VIII metal such as nickel, cobalt, ruthenium, platinum or palladium, as well as copper, zinc, chromium, and mixtures and alloys of these. The preferred catalysts are particulate nickel-based compositions on water-stable (e.g. ceramic) supports, such as are commercially available as Calsicat E-475SR (nickel on a ceramic support, 8x14 mesh spheres) and R-3142 from W.R. Grace. Particle size for the catalyst will be that consistent with fixed-bed operation, which will generally range from 10 microns to 3 mm, with larger particles giving lower pressure drop at the expense of activity.

The invention hydrogenation process can be carried out in one stage or in two or more sequential temperature stages. In a preferred embodiment, hydrogenation is carried out as described above at a temperature within the range of 50 to 130 °C, followed by a second stage carried out at a temperature higher than that of the first stage and within the range of 70 to 155 °C, and then optionally a third stage at a temperature greater than 120 °C for reversion of heavy ends to 1,3-propanediol. In such a process, the illustrated hydrogenation zone 11 includes a series of two or more hydrogenation stages, optionally carried out in two or more separate reaction vessels. Dilution stream 9 is preferably output from the first hydrogenation stage. The preferred catalysts for the second-stage hydrogenation include nickel-based catalysts such as those referenced above for the first stage, as well as copper chromite or copper-zinc catalysts.

Residual solvent and extractant water can be recovered by distillation in column 14 and recycled via 15 to the water extraction process via a further distillation (not shown) for separation and purge of light ends. 1,3-Propanediol-containing product stream 16 can be passed to distillation column 17 for recovery of 1,3-propanediol 18 from heavy ends 19.

### Example 1

A series of hydrogenation experiments was performed in a 0.5-L autoclave reactor with agitation provided by a draft-tube gas dispersion impeller to enhance the rate of transport of hydrogen gas into the liquid phase. Approximately 28g of a commercial 8/14-mesh spherical supported nickel catalyst (50 wt% nickel on silica/-alumina, 0.43 ml/g pore volume) were retained in an annular catalyst basket to examine fixed-bed catalyst performance. The same catalyst was crushed under an inert atmosphere to give 1-20 micron particles for examination of slurry catalyst performance. For slurry studies, the crushed catalyst was added directly to the reactor in the absence of the catalyst basket. The reactor was charged with 300-350 ml of aqueous hydroformylation product rich in 3-hydroxypropanal (HPA) intermediate. The reactor was operated at 1000-1700 rpm agitation and 6.9 MPa (1000 psi) hydrogen, which was replenished as depleted during the reaction. A filtered dip tube allowed sampling of the reactor to monitor the course of reaction. Samples were analyzed using a temperature-programmed capillary gas chromatograph to determine remaining HPA and 1,3-propanediol (PDO) formed.

Apparent selectivities were computed as moles of PDO formed per mole of HPA converted. Apparent selectivities in excess of 100% indicate reversion of heavy ends (formed during ethylene oxide hydroformylation) to PDO.

Runs 1-3 examined slurry catalyst performance. Hydrogenation activities (defined as the pseudo-first-order rate constant for consumption of HPA, assuming a first order dependence upon catalyst concentration) ranged from 96 to 233 l/h/wt.cat, with selectivities in excess of 100% at HPA concentrations up to 22 wt%.

Runs 4-7 examined particulate-form (8x14 mesh) catalyst at HPA concentrations from 18 to 23 wt%. For these runs, hydrogenation activities were reduced more than 10-fold relative to the slurry catalyst hydrogenation. Selectivity for the initial hydrogenation run was only 85%. The observed selectivity and activity diminished upon recycle of the catalyst, indicating degradation of catalyst performance.

Runs 8-11 examined performance of the particulate catalyst over a range of initial HPA concentrations. For these runs, HPA solutions were diluted with deionized water. (Separate runs demonstrated that dilution with PDO/water solutions gave equivalent results to dilution with water alone. Water dilution, however, gave more accurate product analysis than did dilution with PDO/water.) For the initial runs at dilute (2.9-3.3 wt%) HPA concentrations with fresh particulate catalyst (Run 8) and for the first recycle of this catalyst (Run 9), the hydrogenation activity remained essentially constant upon recycle, while selectivity remained at essentially 100%. With an increase in initial HPA concentration to 10.8 wt%, the hydrogenation activity sharply diminished. A further reduction in activity and a decrease in selectivity to 85% were observed upon increasing the initial HPA concentration to 21 wt% in the fourth cycle (Run 11).

For Runs 12-14, initial HPA concentrations were maintained below 4.5 wt%. Observed hydrogenation activities, although diminishing somewhat with catalyst recycle, remained high (at least equal to 50 l/h/wt.cat corrected to 70 °C), and selectivities remained over 100% for all runs.

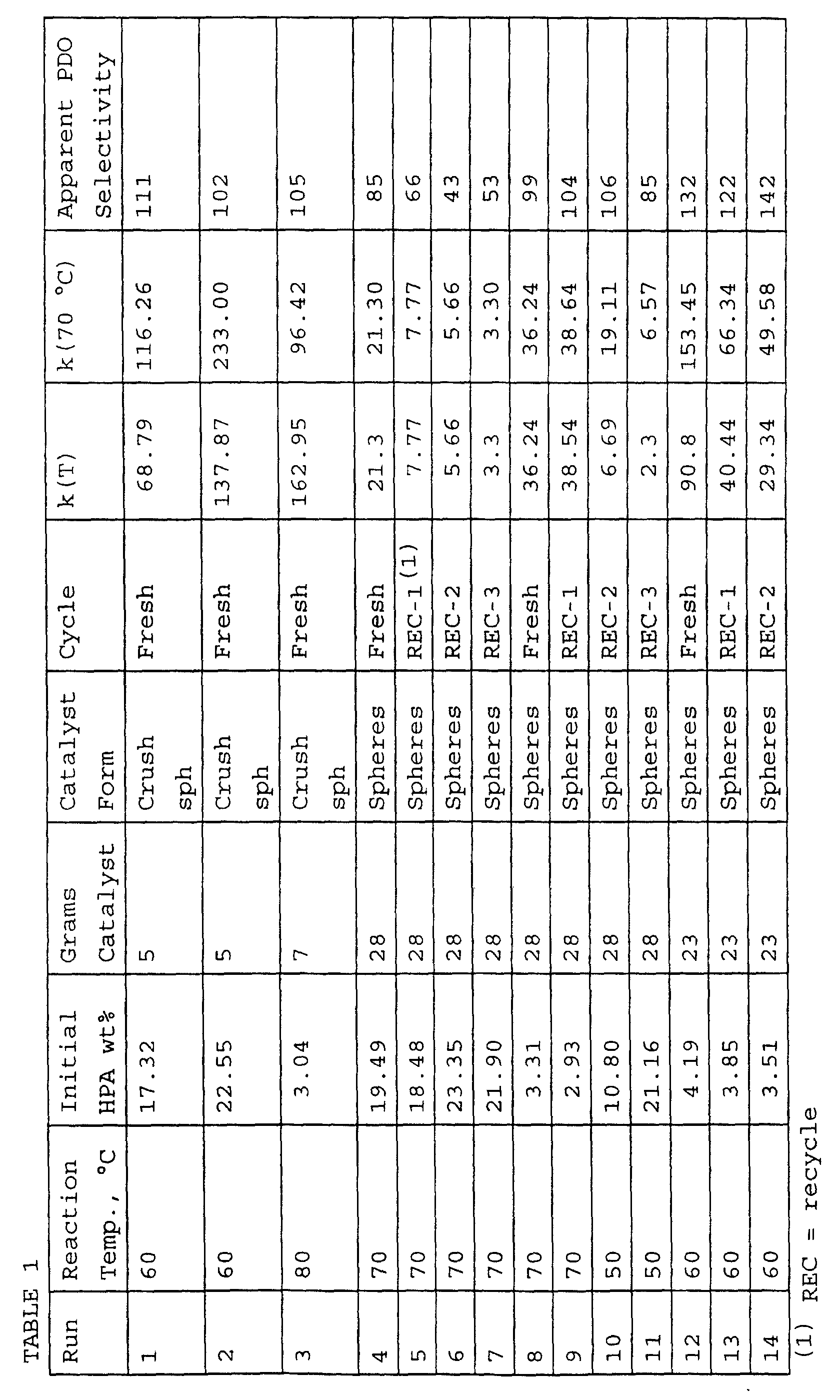

### Example 2

These experiments were performed to determine if an aqueous PDO solution could be used to dilute the hydrogenation input stream without adverse effects on hydrogenation activities.

Two batch hydrogenation reactions were carried out at 70 °C and 6.9 MPa using Calsicat E-475SR hydrogenation catalyst. In run A, a 28 wt% HPA concentrate produced by ethylene oxide hydroformylation and water extraction was diluted with deionized water to give an aqueous solution of 4.8 wt% HPA (with residual 0.2 wt% PDO). In run B, the HPA concentrate was diluted with purified PDO and water to give an aqueous solution of 3.9 wt% HPA and 24.4 wt% PDO. The observed hydrogenation activities were equivalent, within experimental error. Gas chromatographic analysis of product revealed no difference in by-product identity from the two hydrogenation reactions.

### Example 3

A series of experiments was conducted in a 500-ml autoclave reactor containing 28g of the hydrogenation catalyst used in Example 2 and 300-325g of HPA-containing aqueous feed. The aqueous feed was diluted with water to provide aqueous solutions containing between 3.8 and 4.8 wt% HPA. After pressure uptake (70 °C, 6.9 MPa H₂) had been completed, liquid was drained from the reactor and replaced with fresh feed, to perform a recycle test of catalyst performance. Activities and selectivities for 9 recycles conducted in this manner using the diluted HPA solution were compared with those conducted under identical conditions using higher (19.5-21.9 wt%) HPA concentrations.

Use of a diluted HPA solution gave a higher intrinsic catalytic activity which stabilized at a higher value, compared with the recycle reactions using the more concentrated HPA solutions. Selectivity to PDO formation was greatly reduced with the undiluted feed and diminished with time. Excellent selectivities which did not diminish with time were observed with the diluted HPA solutions.

### Example 4

This example illustrates the hydroformylation of ethylene oxide using a cobalt porphyrine-promoted cobalt catalyst, a manganese porphyrine-promoted cobalt catalyst and a ruthenium-promoted cobalt catalyst. 5,10,15,20-Tetraphenyl-21H,23H-porphyrine manganese(III) (manganese TPP), 5,10,15,20-tetraphenyl-21H,23H-porphyrine cobalt(II) (cobalt TPP), triruthenium dodecacarbonyl (Ru₃(CO)₁₂) and dicobalt octacarbonyl (Co₂(CO)₈) were used as the source of the metals. The amount of each catalyst component is listed as mmoles of metal atoms to allow a quantitative comparison on an atomic basis. Reaction times were 1 hour.

Manganese TPP and dicobalt octacarbonyl were introduced to a 100-ml Parr autoclave containing 34 ml of water-saturated (about 2 wt%) nitrogen-purged methyl-t-butyl ether (MTBE). The reactor was pressured with 4.8 MPa (700 psig) of 2:1 H₂/CO. The reaction solution was then heated with stirring, and 1.5g (34 mmoles) of ethylene oxide was charged to the reaction vessel after the reaction temperature had been reached. The pressure was increased to about 11.4 MPa (1500 psig) with additional H₂/CO. Stirring was continued for 1 hour and the reaction mixture was cooled to 5 °C. The gases were slowly vented to ambient pressure and 20 ml of nitrogen-purged deionized water were injected. The reaction mixture was stirred for 5 minutes and the phases were then allowed to separate. The water phase containing the reaction product was removed and analyzed (on the presence of acetaldehyde, "AA"; oligomers; HPA; PDO and other compounds) by gas chromatography. Results are shown in Table 2.

In some instances, the reactor containing the remaining MTBE and catalyst was repressured to 4.8 MPa with H₂/CO and heated to reaction temperature. After 30 minutes, 1.5g of ethylene oxide was again injected and the pressure was increased to about 11.4 MPa with additional H₂/CO. After stirring for 1 hour under reaction conditions, the reaction mixture was cooled, vented to ambient pressure, extracted with deionized water, and phase separated under an inert atmosphere. The aqueous and organic phases were analyzed for product content by gas chromatography. In several instances, the separated phases were also analyzed for metal content. The Experiments using "recycled" catalyst are designated with an "R" in the Table. The percentage of catalyst remaining in the MTBE after the reaction in run 3R amounted to 96.8% on cobalt and 89.0% on manganese.

As can be seen from the results, ruthenium, manganese TPP and cobalt TPP have a promotional effect for the dicobalt octacarbonyl catalyst. The promoters are effective even at very low concentrations.

### Example 5

This example illustrates the hydroformylation of propylene oxide catalyzed by a manganese porphyrine-promoted cobalt catalyst in accordance with the invention process.

A 100-ml Parr autoclave was charged with 115 mg (0.34 mmole) of dicobaltoctacarbonyl, 25 mg (0.037 mmole) of manganese TPP, 34 ml of water-saturated (about 2 wt%) nitrogen-purged MTBE, and 1.98g propylene oxide (34 mmole). The reaction mixture was heated with stirring at 80°C and 10.7 MPa (1400 psig) H₂/CO (2:1) for 1 hour and then cooled to 5 °C. The gases were slowly vented to ambient pressure, and 25 ml of nitrogen-purged, deionized water were injected into the reactor. After stirring for 5 minutes and allowing phase separation, the water phase was removed from the reactor and analyzed by gas chromatography. Selectivities to the organic products were 10.1% acetone, 71.4% 3-hydroxybutyraldehyde, 7.4% 2-methylpropionaldehyde, 6.7% 1,3-butanediol and 4.4% oligomers. Propylene oxide conversion was 60%.

Similar results were achieved with cobalt TPP and triruthenium dodecacarbonyl.

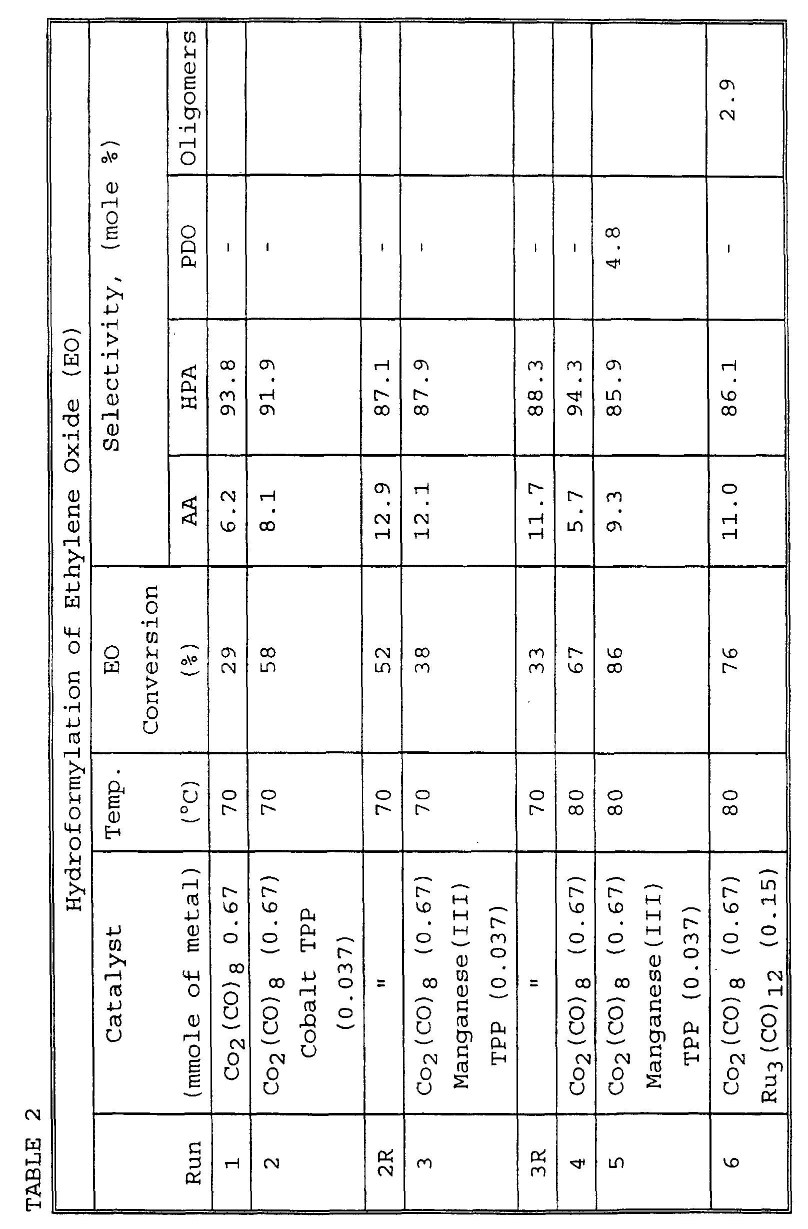

## Claims

1. A process for preparing 1,3-alkanediols via the hydrogenation of 3-hydroxyalkanals wherein the process comprises passing an aqueous 3-hydroxyalkanal solution to a hydrogenation zone and in contact with a fixed-bed hydrogenation catalyst under hydrogenation conditions to form an aqueous 1,3-alkanediol solution, **characterized in that** the aqueous 3-hydroxyalkanal solution has a 3 hydroxyalkanal concentration within the range of 0.2 to 5 wt%.

2. The process of claim 1 in which the aqueous 3-hydroxyalkanal solution is passed to a first hydrogenation reactor and then to a second hydrogenation reactor maintained at a higher temperature than said first hydrogenation reactor.

3. The process of claim 2 in which the first hydrogenation reactor operates at a temperature within the range of 50 to 130 °C, and the second hydrogenation reactor operates at a higher temperature than said first hydrogenation reactor and within the range of 70 to 155 °C.

4. The process of any one of claims 1 to 3 in which the hydrogenation catalyst comprises nickel.

5. The process of claim 4 in which the catalyst comprises nickel on a ceramic support.

6. The process of any one of claims 1 to 5, in which the aqueous 3-hydroxyalkanal solution is prepared by a process comprising the steps of:
(a) contacting, at a temperature within the range of 50 to 100 °C and a pressure within the range of 0.7 to 34.5 MPa, alkylene oxide with carbon monoxide and hydrogen in an essentially non-water-miscible solvent in the presence of an effective amount of a non-phosphine-ligated cobalt or rhodium catalyst and an effective amount of a catalyst promoter under reaction conditions effective to produce an intermediate product mixture comprising less than 15 wt% 3-hydroxyalkanal;
(b) adding an aqueous liquid to said intermediate product mixture and extracting into said aqueous liquid a major portion of the 3-hydroxyalkanal at a temperature less than 100 °C so as to provide an aqueous phase comprising 3-hydroxyalkanal in a concentration of at least 20 wt%, and an organic phase comprising at least a portion of the cobalt or rhodium catalyst or a cobalt- or rhodium-containing derivative thereof;
(c) separating the aqueous phase from the organic phase; and
(d) diluting the aqueous phase comprising 3-hydroxyalkanal with an aqueous liquid to form a solution having a 3-hydroxyalkanal concentration within the range of 0.2 to 5 wt%.

7. The process of claim 6 in which the dilution of step (d) is effected by adding an aqueous solution of 1,3-alkanediol.

8. The process of claim 6, wherein the promoter is a lipophilic ruthenium promoter, or a transition metal porphyrin.

9. The process of any one of claims 1 to 8, wherein 1,3-propanediol is made from 3-hydroxypropanal.

10. A process for preparing 1,3-propanediol comprising the steps of:
(a) contacting ethylene oxide with carbon monoxide and hydrogen in an essentially non-water-miscible solvent in the presence of an effective amount of a non-phosphine-ligated cobalt catalyst and an effective amount of a catalyst promoter under reaction conditions effective to produce an intermediate product mixture comprising less than 15 wt% 3-hydroxypropanal;
(b) adding an aqueous liquid to said intermediate product mixture and extracting into said aqueous liquid a major portion of the 3-hydroxypropanal at a temperature less than 100 °C so as to provide an aqueous phase comprising the 3-hydroxypropanal in greater concentration than the concentration of 3-hydroxypropanal in the intermediate product mixture, and an organic phase comprising at least a portion of the cobalt catalyst or a cobalt-containing derivative thereof;
(c) separating the aqueous phase from the organic phase;
(d) adding to said aqueous phase an aqueous solution of 1,3-propanediol to form a dilute aqueous 3-hydroxypropanal solution having a 3-hydroxypropanal concentration within the range of 0.3 to 4.8 wt%;
(e) passing the dilute 3-hydroxypropanal solution to a hydrogenation zone containing a fixed-bed nickel hydrogenation catalyst maintained under hydrogenation conditions of temperature and pressure and therein converting at least a portion of the 3-hydroxypropanal to 1,3-propanediol; and
(f) recovering the 1,3-propanediol.

## Patentansprüche

1. Verfahren zur Herstellung von 1,3-Alkandiolen über die Hydrierung von 3-Hydroxyalkanalen, worin das Verfahren ein Führen einer wäßrigen 3-Hydroxyalkanallösung zu einer Hydrierzone und ein Kontaktieren mit einem Festbetthydrierkatalysator unter Hydrierbedingungen zur Ausbildung einer wäßrigen 1,3-Alkandiollösung umfaßt, **dadurch gekennzeichnet, daß** die wäßrige 3-Hydroxyalkanallösung eine 3-Hydroxyalkanalkonzentration im Bereich von 0,2 bis 5 Gew.-% aufweist.

2. Verfahren nach Anspruch 1, worin die wäßrige 3-Hydroxyalkanallösung zu einem ersten Hydrierreaktor und dann zu einem zweiten Hydrierreaktor geführt wird, der auf einer höheren Temperatur gehalten wird als der erste Hydrierreaktor.

3. Verfahren nach Anspruch 2, worin der erste Hydrierreaktor bei einer Temperatur im Bereich von 50 bis 130°C betrieben wird und der zweite Hydrierreaktor bei einer höheren Temperatur als der erste Hydrierreaktor und in einem Bereich von 70 bis 155°C betrieben wird.

4. Verfahren nach einem der Ansprüche 1 bis 3, worin der Hydrierkatalysator Nickel umfaßt.

5. Verfahren nach Anspruch 4, worin der Katalysator Nickel auf einem keramischen Träger umfaßt.

6. Verfahren nach einem der Ansprüche 1 bis 5, worin die wäßrige 3-Hydroxyalkanallösung nach einem Verfahren hergestellt wird, das die folgenden Stufen umfaßt:
(a) Inkontaktbringen von Alkylenoxid mit Kohlenmonoxid und Wasserstoff bei einer Temperatur im Bereich von 50 bis 100°C und einem Druck im Bereich von 0,7 bis 34,5 mPa in einem im wesentlichen mit Wasser nicht mischbaren Lösungsmittel in Gegenwart einer wirksamen Menge eines nicht mit Phosphin ligierten Cobalt- oder Rhodiumkatalysators und einer wirksamen Menge eines Katalysatorpromotors unter Reaktionsbedingungen, die zur Ausbildung eines weniger als 15 Gew.-% 3-Hydroxyalkanal umfassenden intermediären Produktgemisches wirksam sind;
(b) Zusetzen einer wäßrigen Flüssigkeit zu dem intermediären Produktgemisch und Extrahieren eines Hauptanteiles des 3-Hydroxyalkanals bei einer Temperatur unter 100°C in die wäßrige Flüssigkeit zur Ausbildung einer wäßrigen Phase, die 3-Hydroxyalkanal in einer Konzentration von wenigstens 20 Gew.-% umfaßt, und einer organischen Phase, die wenigstens einen Teil des Cobalt- oder Rhodiumkatalysators oder eines cobalt- oder rhodiumhältigen Derivats hievon umfaßt;
(c) Abtrennen der wäßrigen Phase von der organischen Phase; und
(d) Verdünnen der 3-Hydroxyalkanal umfassenden wäßrigen Phase mit einer wäßrigen Flüssigkeit zur Ausbildung einer Lösung, die eine 3-Hydroxyalkanalkonzentration im Bereich von 0,2 bis 5 Gew.-% aufweist.

7. Verfahren nach Anspruch 6, worin die Verdünnung von Stufe (d) durch Zusetzen einer wäßrigen Lösung von 1,3-Alkandiol ausgeführt wird.

8. Verfahren nach Anspruch 6, worin der Promotor ein lipophiler Rutheniumpromotor oder ein Übergangsmetallporphyrin ist.

9. Verfahren nach einem der Ansprüche 1 bis 8, worin 1,3-Propandiol aus 3-Hydroxypropanal hergestellt wird.

10. Verfahren zur Herstellung von 1,3-Propandiol, das die folgenden Stufen umfaßt:
(a) Inkontaktbringen von Ethylenoxid mit Kohlenmonoxid und Wasserstoff in einem im wesentlichen mit Wasser nicht mischbaren Lösungsmittel in Gegenwart einer wirksamen Menge eines nicht-phosphinligierten Cobaltkatalysators und einer wirksamen Menge eines Katalysatorpromotors unter Reaktionsbedingungen, die zur Ausbildung eines intermediären Produktgemisches wirksam sind, das weniger als 15 Gew.-% 3-Hydroxypropanal umfaßt;
(b) Zusetzen einer wäßrigen Flüssigkeit zu dem intermediären Produktgemisch und Extrahieren eines Hauptanteils des 3-Hydroxypropanals in diese wäßrige Flüssigkeit bei einer Temperatur unter 100°C, zur Ausbildung einer wäßrigen Phase, die das 3-Hydroxypropanal in größerer Konzentration umfaßt als die Konzentration des 3-Hydroxypropanals in dem intermediären Produktgemisch, sowie einer organischen Phase, die wenigstens einen Teil des Cobaltkatalysators oder eines cobalthältigen Derivats hievon umfaßt;
(c) Abtrennen der wäßrigen Phase von der organischen Phase;
(d) Zusetzen einer wäßrigen Lösung von 1,3-Propandiol zu der wäßrigen Phase zur Ausbildung einer verdünnten wäßrigen 3-Hydroxypropanallösung mit einer 3-Hydroxypropanalkonzentration im Bereich von 0,3 bis 4,8 Gew.-%;
(e) Führen der verdünnten 3-Hydroxypropanallösung zu einer Hydrierzone, die einen Festbettnickelhydrierkatalysator enthält und unter Hydrierbedingungen hinsichtlich Temperatur und Druck gehalten wird, und Umwandeln wenigstens eines Teils des 3-Hydroxypropanals zu 1,3-Propandiol in der Hydrierzone; und
(f) Gewinnen des 1,3-Propandiols.

## Revendications

1. Procédé de préparation de 1,3-alcanediols via l'hydrogénation de 3-hydroxyalcanals, lequel procédé consiste à faire passer une solution de 3-hydroxyalcanal aqueuse dans une zone d'hydrogénation et au contact d'un catalyseur d'hydrogénation à lit fixe sous des conditions d'hydrogénation pour former une solution de 1,3-alcanediol aqueuse, **caractérisé en ce que** la solution de 3-hydroxyalcanal aqueuse a une concentration en 3-hydroxyalcanal allant de 0,2 à 5 % en poids.

2. Procédé suivant la revendication 1, dans lequel la solution de 3-hydroxyalcanal aqueuse est amenée dans un premier réacteur d'hydrogénation et ensuite dans un second réacteur d'hydrogénation maintenu à une température plus élevée que le premier réacteur d'hydrogénation.

3. Procédé suivant la revendication 2, dans lequel le premier réacteur d'hydrogénation fonctionne à une température allant de 50 à 130°C, et le second réacteur d'hydrogénation fonctionne à une température plus élevée que le premier réacteur d'hydrogénation et allant de 70 à 155°C.

4. Procédé suivant l'une quelconque des revendications 1 à 3, dans lequel le catalyseur d'hydrogénation comprend du nickel.

5. Procédé suivant la revendication 4, dans lequel le catalyseur comprend du nickel sur un support céramique.

6. Procédé suivant l'une quelconque des revendications 1 à 5, dans lequel la solution de 3-hydroxyalcanal aqueuse est préparée par un procédé comprenant les étapes suivantes
(a) la mise en contact, à une température allant de 50 à 100°C et à une pression allant de 0,7 à 34,5 MPa, d'oxyde d'éthylène avec du monoxyde de carbone et de l'hydrogène dans un solvant essentiellement non miscible à l'eau en présence d'une quantité efficace d'un catalyseur au cobalt ou rhodium lié par coordination avec une non-phosphine et d'une quantité efficace d'un promoteur de catalyseur sous des conditions de réaction efficaces pour produire un mélange de produits intermédiaires comprenant moins de 15 % en poids de 3-hydroxyalcanal;
(b) l'addition d'un liquide aqueux au mélange de produits intermédiaires précité et l'extraction dans ledit liquide aqueux d'une portion majeure du 3-hydroxyalcanal à une température inférieure à 100°C de manière à obtenir une phase aqueuse comprenant du 3-hydroxyalcanal en une concentration d'au moins 20 % en poids, et une phase organique comprenant au moins une partie du catalyseur au cobalt ou rhodium ou d'un dérivé contenant du cobalt ou du rhodium de celui-ci;
(c) la séparation de la phase aqueuse de la phase organique;
(d) la dilution de la phase aqueuse comprenant du 3-hydroxyalcanal avec un liquide aqueux pour former une solution ayant une concentration en 3-hydroxyalcanal allant de 0,2 à 5 % en poids.

7. Procédé suivant la revendication 6, dans lequel la dilution de l'étape (d) est effectuée en ajoutant une solution aqueuse de 1,3-alcanediol.

8. Procédé suivant la revendication 6, dans lequel le promoteur est un promoteur au ruthénium lipophile ou une porphyrine de métal de transition.

9. Procédé suivant l'une quelconque des revendications 1 à 8, dans lequel du 1,3-propanediol est obtenu à partir de 3-hydroxypropanal.

10. Procédé de préparation de 1,3-propanediol comprenant les étapes suivantes :
(a) la mise en contact d'oxyde d'éthylène avec du monoxyde de carbone et de l'hydrogène dans un solvant essentiellement non miscible à l'eau en présence d'une quantité efficace d'un catalyseur au cobalt lié par coordination avec une non-phosphine et d'une quantité efficace d'un promoteur de catalyseur sous des conditions de réaction efficaces pour obtenir un mélange de produits intermédiaires comprenant moins de 15 % en poids de 3-hydroxypropanal;
(b) l'addition d'un liquide aqueux au mélange de produits intermédiaires précité et l'extraction dans ledit liquide aqueux d'une portion majeure du 3-hydroxypropanal à une température inférieure à 100°C de manière à obtenir une phase aqueuse comprenant le 3-hydroxypropanal en une concentration plus élevée que la concentration de 3-hydroxypropanal dans le mélange de produits intermédiaires, et une phase organique comprenant au moins une partie du catalyseur au cobalt ou d'un dérivé contenant du cobalt de celui-ci;
(c) la séparation de la phase aqueuse de la phase organique;
(d) l'addition à la phase aqueuse d'une solution aqueuse de 1,3-propanediol pour former une solution de 3-hydroxypropanal aqueuse diluée ayant une concentration en 3-hydroxypropanal allant de 0,3 à 4,8 % en poids;
(e) le passage de la solution de 3-hydroxypropanal diluée dans une zone d'hydrogénation contenant un catalyseur d'hydrogénation au nickel à lit fixe maintenu sous des conditions d'hydrogénation de température et de pression et la conversion d'au moins une partie du 3-hydroxypropanal en 1,3-propanediol; et
(f) la récupération du 1,3-propanediol.
